# EUROPEAN PATENT APPLICATION

(11) **EP 4 019 134 A1**
(43) Date of publication of application: **29.06.2022**
(21) Application number: 21216263.0
(22) Date of filing: 21.12.2021
(51) Int. Cl.: B01L 3/00

(54) **MICROFLUIDIC DEVICE UNIT**

(30) Priority: 23.12.2020 EP 20217158
(71) Applicant: Imec VZW, 3001 Leuven (BE)
(72) Inventor: JONES, Benjamin, 3010 Kessel-Lo (BE); CHOE, Young Jae, 1040 Etterbeek (BE)
(74) Representative: DenK iP bv

(57) **Abstract**

A microfluidic device unit (1) comprising: (a) a unit inlet (10) and a unit outlet (14), (b) a cavity (11) comprising a fluidic channel (112), (c) a fluidic resistor (12), and (d) a filter (130), wherein the unit inlet (10), the unit outlet (14), the fluidic channel (112), and the fluidic resistor (12) are fluidically coupled to one another, wherein the cavity (11), the fluidic resistor (12), and the filter (13) are between the unit inlet (10) and the unit outlet (14), wherein the cavity (11) is upstream of the fluidic resistor (12), and wherein the filter (13) is positioned so as to filter fluid after it enters the fluidic channel (112) and before it enters the fluidic resistor (12).

## Description

### Technical field of the invention

The present invention relates to the field of microfluidic devices for sensing applications. More specifically, the present invention relates to a microfluidic device unit and a microfluidic device array comprising the same, a process for forming the microfluidic device array, and a method for sensing an analyte making use of the microfluidic device array.

### Background of the invention

A microfluidic device array comprising a plurality of microfluidic device units may be used for instance for sensing of an analyte or for combinational chemistry. Each of the plurality of microfluidic device units is fluidically coupled to an array inlet channel and an array outlet channel of the microfluidic device array. Thereby, upon introduction of a fluid in the array inlet channel of the microfluidic device array, a fraction of the fluid may be introduced in each of the microfluidic device units of the microfluidic device array.

For instance, when the microfluidic device array is used for sensing, the microfluidic device unit may comprise a probe for interacting with the analyte: the interacting preferably results in a detectable signal, such as a shift in fluorescence emitted by the probe. Advantageously, each of the plurality of microfluidic device units may comprise a different probe, wherein each of the different probes is suitable for interacting with a different analyte. Thereby, the microfluidic device array may enable parallel detection of a plurality of analytes in a fluid e.g. sample comprising a plurality of analytes. In another example, the microfluidic device array may be used for combinational chemistry, to prepare, in parallel, a large number of compounds, such as has been for instance described in X. Zhou et al., Nucleic Acids Research 32 (2004) pages 5409-5417.

In the state of the art, the microfluidic device unit usually comprises a fluidic resistor for reducing a flow rate of the fluid through the microfluidic device unit, by increasing a pressure drop across the microfluidic device unit. Thereby, as a fluidic resistance of the microfluidic device unit is larger than a fluidic resistance of the array inlet channel and the array outlet channel of the microfluidic device array, a flow rate of the fluid through the microfluidic device array is limited by the flow rate of the fluid through the microfluidic device unit. As a result, the fluidic resistor facilitates uniformity in the flow rate among the plurality of microfluidic device units of the microfluidic device array. Thereby, for instance, if a reaction in the cavity of the microfluidic device unit (e.g. between an analyte in the fluid and a probe in the cavity) is flow rate dependent, the uniform flow rate ensures a uniform and controllable reaction rate among the plurality of microfluidic device units. Furthermore, the uniform flow rate through each of the plurality of microfluidic device units may advantageously facilitate the removal of air bubbles present in the microfluidic device array. Although the air bubbles may be removed by using a very high flow rate, the very high flow rate may induce any material comprised in the cavity to be removed as well. Finally, a uniform flow rate for each of the microfluidic device units, which implies a small pressure difference between adjacent microfluidic device units, may prevent fluid from flowing between the microfluidic device units, thereby preventing for instance material from moving between the microfluidic device units.

The fluidic resistor usually comprises a trench with a small width e.g. a few micrometer or smaller, wherein the small width provides the fluidic resistance: the smaller the width of the fluidic resistor, the larger the fluidic resistance of the fluidic resistor. In the state of the art, when the width becomes too small, the fluidic resistor may become clogged, by e.g. particles that may be present in the fluid or the microfluidic device unit. For instance, the particles may be introduced during manufacture or spotting of material e.g. the probe in the microfluidic device unit, or may be present in the material, and may partially or completely block the fluid from moving through the fluidic resistor. As the width of the fluidic resistor may not be too small, in the state of the art, the fluidic resistance that a fluidic resistor may have remains limited. Nevertheless, to ensure a uniform flow rate, a large fluidic resistance is highly preferred.

There is a need in the state of the art for enabling reliable and long-lasting high fluidic resistance in fluidic resistors of microfluidic devices.

### Summary of the invention

It is an object of the present invention to provide a reliable microfluidic device unit, a microfluidic device array comprising a plurality of the microfluidic device units, and a process for making the microfluidic device array. The above objective is accomplished by a method and device according to the present invention.

It is an advantage of embodiments of the present invention that each microfluidic device unit comprises a fluidic resistor, which may have a large fluidic resistance, so that a microfluidic device array comprising a plurality of the microfluidic device units may have a uniform flow rate through each of the plurality of the microfluidic device units. It is a limitation of prior art that the cross-sectional dimensions of the fluidic resistor must be relatively large to prevent clogging; thus, the fluidic resistance added by the fluidic resistor is relatively small in the prior art. It is an advantage of embodiments of the present invention that the cross-sectional dimensions are not limited by the risk of clogging and thus, the fluidic resistance of the fluidic resistor may be much larger. It is an advantage of embodiments of the present invention that clogging of the fluidic resistor, for instance by particles, may be prevented. It is an advantage of embodiments of the present invention that particles present in the microfluidic device unit may not be able to clog the fluidic resistor by blocking fluid from flowing through the fluid resistor. It is an advantage of embodiments of the present invention that the microfluidic device unit may have a smaller chance of malfunction due to the clogging.

In a first aspect, the present invention relates to a microfluidic device unit comprising: (a) a unit inlet and a unit outlet, (b) a cavity comprising a fluidic channel, (c) a fluidic resistor, and (d) a filter, wherein the unit inlet, the unit outlet, the fluidic channel, and the fluidic resistor are fluidically coupled to one another, wherein the cavity, the fluidic resistor, and the filter are between the unit inlet and the unit outlet, wherein the cavity is upstream of the fluidic resistor, and wherein the filter is positioned so as to filter fluid after it enters the fluidic channel and before it enters the fluidic resistor.

In a second aspect, the present invention relates to a microfluidic device array comprising (a) an array inlet channel and an array outlet channel, and (b) a plurality of microfluidic device units according to embodiments of the first aspect of the present invention, wherein the unit inlet of each of the microfluidic device units is fluidically coupled to the array inlet channel, and wherein the unit outlet of each of the microfluidic device units is fluidically coupled to the array outlet channel.

In a third aspect, the present invention relates to a method for sensing an analyte, comprising: (a) obtaining a microfluidic device array according to embodiments of the second aspect of the present invention, (b) introducing, via the array inlet channel of the microfluidic device array, a fluid comprising an analyte, and (c) detecting a response of a probe, comprised in the cavity of a microfluidic device unit comprised in the microfluidic device array, to the analyte.

In a fourth aspect, the present invention relates to a process for manufacturing a microfluidic device array according to embodiments of the second aspect of the present invention, comprising the steps of: (a) providing a substrate comprising the plurality of microfluidic device units, (b) providing a cover, (c) providing an array inlet channel and an array outlet channel, and (d) covering the substrate, wherein the microfluidic device array is arranged so that the unit inlet of each of the microfluidic device units is fluidically coupled to the array inlet channel, and that the unit outlet of each of the microfluidic device units is fluidically coupled to the array outlet channel.

In a fifth aspect, the present invention relates to a diagnostic apparatus comprising the microfluidic device array according to embodiments of the second aspect of the present invention.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable, and reliable devices of this nature.

The above and other characteristics, features, and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic horizontal cross-section of a microfluidic device unit according to an embodiment of the present invention.
FIG. 2 is a schematic vertical cross-section of part of a microfluidic device unit according to an embodiment of the present invention.
FIG. 3 is a diagrammatic illustration of a microfluidic device array 3 according to an embodiment of the present invention

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third, and the like in the description and in the claims, are used for distinguishing between similar elements and not necessarily for describing a sequence, either temporally, spatially, in ranking, or in any other manner. It is to be understood that the terms so used are interchangeable under appropriate circumstances and that the embodiments of the invention described herein are capable of operation in other sequences than described or illustrated herein.

Moreover, the terms top, bottom, over, under, and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a microfluidic device unit comprising: (a) a unit inlet and a unit outlet, (b) a cavity comprising a fluidic channel, (c) a fluidic resistor, and (d) a filter, wherein the unit inlet, the unit outlet, the fluidic channel, and the fluidic resistor are fluidically coupled to one another, wherein the cavity, the fluidic resistor, and the filter are between the unit inlet and the unit outlet, wherein the cavity is upstream of the fluidic resistor, and wherein the filter is positioned so as to filter fluid after it enters the fluidic channel and before it enters the fluidic resistor.

Any features of any embodiment of the first aspect may be independently as correspondingly described for any embodiment of any of the other aspects of the present invention.

In embodiments, the unit inlet is for introducing a fluid into the microfluidic device unit e.g. into the cavity. The fluid may for instance comprise a liquid, a gas, a vapor, or an aerosol. In embodiments, a flow path of a fluid introduced in the microfluidic device unit is from the unit inlet, through the fluidic channel of the cavity, through the fluidic resistor, and to the unit outlet. In embodiments, the cavity being located upstream of the fluidic resistor, the fluid flows first through the cavity and then through the fluidic resistor.

In embodiments, each of the unit inlet, the unit outlet, and the fluidic channel comprises a trench. However, the invention is not limited thereto, and instead, the unit inlet, the unit outlet, and the fluidic channel may for instance each comprise a tube. In other embodiments, one or more of the unit inlet, unit outlet, and the fluidic channel may comprise a trench while the others may comprise a tube. In embodiments, a width of the unit inlet may be from 10 µm to 1 mm, such as from 50 µm to 200 µm. In embodiments, the width of the unit inlet is equal to or smaller than a width of the fluidic channel of the cavity. In embodiments, a height of the unit inlet may be from 10 µm to 1 mm, such as from 50 µm to 200 µm. In embodiments, the unit outlet is for removing the fluid from the microfluidic device unit e.g. from the cavity. In embodiments, a width of the unit outlet may be from 10 µm to 1 mm, such as from 50 µm to 200 µm. In embodiments, the width of the unit outlet is equal to or smaller than the width of the fluidic channel of the cavity. In embodiments, a height of the unit outlet may be from 10 µm to 1 mm, such as from 10 µm to 200 µm. In preferred embodiments, the height of the unit outlet is larger than a height of the fluidic resistor. In preferred embodiments, the width of the unit outlet is larger than the width of a fluidic resistor. Advantageously, when the unit outlet has larger dimensions than the fluidic resistor, air bubbles may be more easily removed from the microfluidic device unit. In embodiments, the fluidic channel has a width of from 10 µm to 1 mm, such as from 10 µm to 200 µm. In embodiments, a height of the unit inlet may be from 10 µm to 1 mm, such as from 10 µm to 200 µm. Preferably, the unit inlet, the unit outlet, and the fluidic channel have dimensions such that a flow rate of the fluid is not limited by the unit inlet, the unit outlet, or the fluidic channel. In preferred embodiments, the unit outlet has a larger height and a larger width than the unit inlet. Advantageously, in these embodiments, air bubbles may be more easily removed from the microfluidic device unit.

In embodiments, the fluidic resistance of the fluidic resistor is larger, preferably at least ten times larger, more preferably at least a hundred times larger, than the fluidic resistance of each of the unit inlet, of the unit outlet, and of the fluidic channel. Thereby, the flow rate of the fluid through the microfluidic device unit may be limited by the flow rate of the fluid through the fluidic resistor. In embodiments, the fluidic resistance of a channel, e.g. the unit inlet, the unit outlet, the fluidic channel, or the fluidic resistor, with a rectangular cross-section, and a width w smaller than a height h and a length L, is proportional to L/(hw³×(1-0.630w/h)); when the width is much smaller than, e.g. negligible compared to, the height and the length, the proportionality simplifies to L/(hw³). In embodiments, the fluidic resistance of a channel of length L and with a circular cross-section with diameter d is proportional to L/d⁴. Hence, in embodiments, the fluidic resistance may be increased by increasing the length of the fluidic resistor. In embodiments, the fluidic resistor comprises a trench with a width that is at least ten times, such as at least a hundred times smaller than a height. Although, alternatively, the fluidic resistor may e.g. comprise a cylindrical shape, the trench is preferred as it is easier to manufacture. In embodiments, the fluidic resistor has a width that is smaller than the width of each of the unit inlet, the unit outlet, and the fluidic channel. In embodiments, the fluidic resistor has a width at least ten times smaller than the width of the fluidic channel. In embodiments, the fluidic resistor has a width of from 1 to 20 µm, preferably from 2 to 10 µm. In embodiments, the fluidic resistor is straight. In different embodiments, the fluidic resistor is curved or meandrous. Advantageously, in these embodiments, for a same distance between a fluidic resistor inlet and a fluidic resistor outlet, the length of the fluidic resistor may be longer than when the fluidic resistor is straight. This may for instance be preferred when a large fluidic resistance (and hence length of the fluidic resistor) is required, but space within the microfluidic device unit is limited. In embodiments, the length of the fluidic resistor is from 10 µm to 1 mm. In embodiments, the fluidic resistor has a height of from 1 µm to 1 mm, such as from 10 µm to 200 µm.

In embodiments, the filter comprises a plurality of micropillars, such as at least five micropillars, wherein the distance between neighbouring micropillars, i.e. a width of a gap between neighbouring micropillars, is less than or equal to the width of the fluidic resistor and preferably between 50 to 80% of the width of the fluidic resistor. Thereby, material e.g. particles that cannot pass through the fluidic resistor, i.e. that would clog the fluidic resistor, can also not pass through the filter. In embodiments, the distance between neighbouring micropillars is uniform, that is, within 20%, such as within 10%, preferably within 2%, of a mean distance between neighbouring micropillars among the plurality of micropillars of the filter. In embodiments, the width of the filter equals the width of the fluidic channel. In embodiments, the fluid cannot flow to the fluidic resistor without being filtered by the filter. In embodiments, the long axis of each of the plurality of micropillars is aligned within 10° of a same axis, such as within 2° of a same axis, such as parallel to each other. The filter is positioned so as to filter fluid after it enters the fluidic channel and before it enters the fluidic resistor. The filter may, for instance, be positioned in the cavity e.g. the fluidic channel, or between the cavity e.g. fluidic channel and the fluidic resistor. In embodiments, the filter is positioned such that any material e.g. particles in the cavity dragged along by the fluid are filtered out of the fluid by the filter before the fluid may enter the fluidic resistor, so that, in the presence of the filter, clogging of the fluidic resistor may be less likely to occur. Advantageously, in embodiments where the filter comprises a plurality of micropillars, and hence a plurality of gaps between the micropillars, although clogging of some of the gaps may be likely to occur, clogging of each of the gaps, i.e. clogging of the filter, is less likely to occur. Thereby, advantageously, the filter may ensure a continuous flow rate through the microfluidic device unit e.g. over the course of a use of the microfluidic device unit. In embodiments, the micropillars may have any shape, such as cylindrical, rectangular cuboid, or irregular, preferably cylindrical. In embodiments, the width, e.g. the diameter, of the micropillars is from 1 to 100 µm, preferably 1 to 20 µm. Advantageously, a uniform distance between neighbouring micropillars may yield a predictable filtering of the fluid by the filter.

In embodiments, the microfluidic unit may be adapted for detecting an analyte, e.g. for sensing of an odor or a bio-molecule. In embodiments, the cavity comprises a probe for interacting with an analyte. In embodiments, the analyte is comprised in the fluid. When the fluid is introduced in the cavity, the analyte may interact with the probe. In embodiments, the interacting of the probe with the analyte may be detectable. In embodiments, the probe is capable of emitting light after interaction with the analyte. For instance, the probe may, on interaction with the analyte and on illumination, luminesce, e.g. fluoresce or phosphoresce. In embodiments, the probe luminesces also in absence of the interaction with the probe, but for instance at a different wavelength, that is, the interaction induces a shift of the luminescence of the probe. In embodiments, the microfluidic device unit may be used for DNA analysis, wherein an oligonucleotide, that is the probe, is present in the microfluidic device unit, e.g. in the cavity such as in the well of the microfluidic device unit, for binding to specific sequences of the DNA. In these embodiments, the oligonucleotide may comprise a fluorescence tag. The oligonucleotide may bind to the DNA, which may then be observed by visualization of the oligonucleotide to the DNA using an imaging technique.

In embodiments, a wall of the cavity comprises an optical window. Thereby, the cavity may be optically coupled to a location outside of the microfluidic device unit, through the optical window. Advantageously, a probe in the cavity may be illuminated through the optical window by a light source e.g. by a laser, LED or light bulb. At the same time, luminescence emitted by the probe may propagate through the optical window and be detected by a light sensor outside of the microfluidic device unit. The optical window may comprise any transparent material e.g. silicate glass.

In embodiments, the cavity comprises a well fluidically coupled to the fluidic channel, wherein the filter is positioned after the well and before the fluidic resistor. Advantageously, the well may comprise a material. If the microfluidic device unit is used for sensing of an analyte, the material may comprise the probe, but the present invention is not limited thereto. For instance, if the microfluidic device unit is used for synthesis, the material may be for initiating or promoting the synthesis. Advantageously, the well may define an area for introducing the material in the cavity, e.g. via spotting, for instance using a robotic arm. The well may for instance comprise a recess in a wall of the fluidic channel. In embodiments, the well has a cylindrical or a rectangular cuboid shape, although the present invention is not limited to any shape, and instead, the well may alternatively have an irregular shape. Thereby, advantageously, any material that is in the well may not be moved by the fluid flowing from the unit inlet to the unit outlet through the fluidic channel. In embodiments, the well may have a width that is smaller than the width of the fluidic channel. In embodiments, the well has a width of from 10 µm to 1 mm. In embodiments, the well may have a depth that is smaller than 1 mm, such as from 50 µm to 500 µm. In embodiments, a volume of the well is from 100 pL to 1 µL. However, although the well has advantages, the well is not essential for the present invention, as the material may for instance instead be introduced in the fluidic channel of the cavity.

In a second aspect, the present invention relates to a microfluidic device array comprising (a) an array inlet channel and an array outlet channel, and (b) a plurality of microfluidic device units according to embodiments of the first aspect of the present invention, wherein the unit inlet of each of the microfluidic device units is fluidically coupled to the array inlet channel, and wherein the unit outlet of each of the microfluidic device units is fluidically coupled to the array outlet channel.

Any features of any embodiment of the second aspect may be independently as correspondingly described for any embodiment of any of the other aspects of the present invention.

As the array inlet channel is fluidically coupled to the unit inlet of each of the plurality of microfluidic device units, fluid that is introduced in the array inlet channel may subsequently be introduced in each of the plurality of microfluidic device units. Advantageously, each of the plurality of microfluidic device units comprises a fluidic resistor. In preferred embodiments, a flow rate of the fluid through the microfluidic device array is limited by a flow rate of the fluid through the fluidic resistors. In preferred embodiments, the fluidic resistors of the plurality of microfluidic device units have a fluidic resistance that is within 25% of a mean fluidic resistance among the fluidic resistors of the microfluidic device array. Advantageously, thereby, the fluidic resistors may ensure a uniform flow rate of the fluid through each of the plurality of microfluidic device units. Thereby, for instance, if a reaction in the cavity of the microfluidic device unit e.g. between an analyte in the fluid and a probe in the cavity is flow rate dependent, the uniform flow rate ensures a uniform reaction rate among the plurality of microfluidic device units. Furthermore, the uniform flow rate through each of the plurality of microfluidic device units may advantageously facilitate the removal of air bubbles present in the microfluidic device array. If an air bubble is present in a microfluidic device unit, the air bubble may induce a fluidic resistance within the microfluidic device unit, thereby reducing e.g. preventing fluid flow through the microfluidic device unit. Furthermore, as the fluid flow is reduced, the air bubble may also not be removed from the microfluidic device unit. If the fluidic resistor of the microfluidic device unit has a fluidic resistance that is larger than the fluidic resistance induced by the air bubble, a pressure acting on the air bubble may be large enough to remove the air bubble from the microfluidic device unit.

In embodiments, a width of the array inlet channel may be from 10 µm to 1 mm, such as from 50 µm to 200 µm. In embodiments, a height of the array inlet channel may be from 10 µm to 1 mm, such as from 50 µm to 200 µm. In embodiments, a width of the array outlet channel may be from 10 µm to 1 mm, such as from 50 µm to 200 µm. In embodiments, a height of the array outlet channel may be from 10 µm to 1 mm, such as from 50 µm to 200 µm. In preferred embodiments, the unit inlet has a larger height and a larger width than the array inlet channel. In preferred embodiments, the array outlet channel has a larger height and a larger width than the unit outlet. Advantageously, in these embodiments, air bubbles may be more easily removed from the microfluidic device array.

Advantageously, the filter present in each of the plurality of microfluidic device unit may ensure that the fluidic resistor does not become clogged, thereby further ensuring a uniform flow rate through each of the plurality of microfluidic device units. It is an advantage of embodiments of the present invention that the flow rate through each of the plurality of microfluidic device units of the microfluidic device array remains uniform. Furthermore, a possible failure by the clogging of microfluidic device units of the microfluidic device array may be prevented by the filter.

Alternatively, the microfluidic device array may for instance be used for the parallel synthesis of molecules, such as for the parallel synthesis of different molecules, wherein, for instance, each of the plurality of microfluidic device units is used for the synthesis of a different molecule. For this, for instance, in each of the microfluidic device units, a different primer or initiator may be present.

In a third aspect, the present invention relates to a method for sensing an analyte, comprising: (a) obtaining a microfluidic device array according to embodiments of the second aspect of the present invention, (b) introducing, via the array inlet channel of the microfluidic device array, a fluid comprising an analyte, and (c) detecting a response of a probe, comprised in the cavity of a microfluidic device unit comprised in the microfluidic device array, to the analyte.

Any features of any embodiment of the third aspect may be independently as correspondingly described for any embodiment of any of the other aspects of the present invention.

As the microfluidic device array comprises a plurality of microfluidic device units, the microfluidic device array may for instance be highly suitable for parallel detection of a range of analytes, that is, wherein each of the plurality of microfluidic device units comprises a different probe. In embodiments, detecting a response comprises detecting luminescence emitted by the probe. The detecting may for instance comprise exciting the probe with light, and detecting the luminescence subsequently emitted by the probe. In embodiments, the fluid may comprise a plurality of analytes.

In embodiments, introducing a fluid comprises introducing e.g. injecting the fluid into the array inlet channel of the microfluidic device array, and inducing a flow of the fluid through the array inlet channel, and into each of the plurality of microfluidic device units. In embodiments, fluid may be introduced in the array inlet channel via a microfluidic device array inlet. In embodiments, the fluid is induced to flow through each of the plurality of microfluidic device units i.e. through the unit inlet, through the fluidic channel, and through the unit outlet, into the array outlet channel of the microfluidic device array. In embodiments, fluid may be removed from the array outlet channel via a microfluidic device array outlet. In embodiments, the fluid is induced to flow during the detecting. In embodiments, the fluid is induced to flow continuously from the array inlet channel, through each of the plurality of microfluidic device units, to the array outlet channel during the detecting. Advantageously, each of the plurality of microfluidic device units may comprise a different probe each adapted for interacting with a different analyte, which allows for the parallel detection of a plurality of analytes possibly comprised in the fluid.

In preferred embodiments, the fluid comprises an odor, wherein the odor is for instance comprised in a gas, a vapor, or an aerosol. Odors may comprise a plurality of compounds i.e. analytes, which may be detected in parallel by the microfluidic device array according to embodiments of the present invention. Indeed, each of the plurality of microfluidic device units may comprise a probe for a different compound of the odor, so that, by introducing the odor in the microfluidic device array, each of the plurality of compounds of the odor may be detected in parallel, that is, at the same time. However, the fluid comprising the analyte is not limited to odors. For instance, the microfluidic device array may be used for DNA analysis, that is, wherein the fluid comprises DNA, wherein a wide range of sequences in the DNA may be detected in parallel. Herein, each of the plurality of microfluidic device units may comprise a different oligonucleotide, possibly comprising a luminescent tag. Parallel DNA analysis may be advantageously used for instance for DNA mapping or DNA fingerprinting.

As an example, in the context of DNA detection microarray, in one application of the present invention, a probe can be a first single-stranded DNA molecule of a particular, known sequence with intercalating fluorescent molecules. The intercalating fluorescent molecules fluoresce weakly when bound to a single-stranded DNA molecule but fluoresce strongly when bound to a double-stranded DNA molecule. The probe is immobilized or bound to the surface of the fluidic channel. The analyte, in this example, may be a second, unknown, single-stranded DNA molecule with a sequence different than the first, immobilized, single-stranded DNA molecule. When the fluid containing the analyte is flushed into the fluidic channel, the first and second DNA molecules will interact only if their sequence is complementary. If the sequence is complementary, the two single-stranded DNA molecules will bind to form a double-stranded DNA molecule. Upon excitation by a light source, the intercalating dye will fluoresce strongly only if a double-stranded DNA molecule is formed. Since the first single-stranded DNA molecule sequence is known a *priori,* the sequence of the DNA molecule in the analyte is known if it binds to the first single-stranded DNA molecule as it must be complementary to the first. Thus, the detection of a fluorescent signal allows determination of whether the DNA in the analyte is complementary to the immobilized DNA molecule.

The probe is not limited to single-stranded DNA molecules with intercalating fluorescent molecules. It is understood by someone skilled in the art that many other examples of a probe exist and that applications of the present invention are not limited to the example provided.

In a fourth aspect, the present invention relates to a process for manufacturing a microfluidic device array according to embodiments of the second aspect of the present invention, comprising the steps of: (a) providing a substrate comprising the plurality of microfluidic device units, (b) providing a cover, (c) providing an array inlet channel and an array outlet channel, and (d) covering the substrate with the cover, wherein the microfluidic device array is arranged so that the unit inlet of each of the microfluidic device units is fluidically coupled to the array inlet channel, and that the unit outlet of each of the microfluidic device units is fluidically coupled to the array outlet channel.

Any features of any embodiment of the fourth aspect may be independently as correspondingly described for any embodiment of any of the other aspects of the present invention.

In embodiments, the cover comprises an optical window, for instance comprising silicate glass, sapphire, or a polymeric material. In embodiments, the cover consists of silicate glass. In embodiments wherein a wall of each of the plurality of microfluidic device units of the microfluidic device array comprises an optical window, the wall is comprised in the cover. However, the wall comprising the optical window may in different embodiment not be comprised in the cover. In embodiments, the wall comprising the optical window is comprised in the substrate. An advantage of using silicate glass for the cover is that silicate glass is relatively cheap and straightforward to manufacture.

In embodiments, the array inlet channel and the array outlet channel are comprised in the cover. In these embodiments, the covering of the substrate with the cover is performed so that the array inlet channel in the cover is fluidically couplet to the unit inlet of each of the microfluidic device units in the substrate. Furthermore, in these embodiments, the covering of the substrate with the cover is performed so that the array outlet channel in the cover is fluidically couplet to the unit outlet of each of the microfluidic device units in the substrate. In different embodiments, the array inlet channel and the array outlet channel are comprised in the substrate. In embodiments, the array inlet channel and the array outlet channel may be obtained using an etching technique such as wet etching or reactive ion etching.

In embodiments, the substrate comprises, preferably consists of, silicon or silicate glass. In these embodiments, the filter may comprise, preferably consist of, silicon. In embodiments, the plurality of microfluidic device units may be obtained in the substrate by any suitable technique, such as a patterning technique such as etching e.g. photolithography. Advantageously, in these embodiments, silicon wafers may be used for manufacturing of the substrate, wherein each component of the microfluidic device units is obtained in the substrate by patterning e.g. by etching. This may enable large-scale fabrication of the microfluidic device array according to embodiments of the present invention.

The substrate and the cover may be bonded together using, for example, anodic bonding or fusion bonding.

In embodiments, the process comprises a further step b', after step a and before step c, of spotting material e.g. a probe in at least one microfluidic device unit, such as in a plurality of microfluidic device units comprised in the substrate. In embodiments, step b' may be performed before step b or after step b. In embodiments, the material is spotted in a cavity of the microfluidic device unit, e.g. in a well of each of the cavities. The material may for instance comprise a probe for detecting analytes in a fluid in the microfluidic device unit, or for instance an initiator for initiating a reaction in the microfluidic device unit. In embodiments, the microfluidic device array is sealed by the covering of the substrate with the cover, except for an entrance to the array inlet channel and to the array outlet channel in the cover are exposed e.g. for introducing the fluid into the microfluidic device array.

In particular embodiments, each microfluidic device unit remains exposed after covering the substrate with the cover. Thereby, the material may be spotted into the microfluidic device units after manufacturing of the microfluidic device array. In embodiments, the process comprises a further step c', after step c, of spotting the material in at least one microfluidic device unit, such as in a plurality of microfluidic device units comprised in the substrate. In embodiments, the process comprises a step d after step c' of covering the substrate, at a different side than the side covered by the cover, with a sealing plate, which may for instance comprise silicate glass.

In a fifth aspect, the present invention relates to a diagnostic apparatus comprising the microfluidic device array according to embodiments of the second aspect of the present invention.

Any features of any embodiment of the fifth aspect may be independently as correspondingly described for any embodiment of any of the other aspects of the present invention.

In embodiments, the diagnostic apparatus comprises a light source e.g. for illuminating a probe comprised in the microfluidic device units of the microfluidic device array. In embodiments, the diagnostic apparatus comprises a detector for detecting luminescence emitted by the probe comprised in the microfluidic device units of the microfluidic device array. In embodiments, the diagnostic apparatus comprises a pump for inducing a flow of a fluid through the microfluidic device array. In embodiments, the diagnostic apparatus comprises a reservoir fluidically coupled to the array inlet channel of the microfluidic device array. The reservoir may comprise a fluid that is to be introduced in the microfluidic device array. For example, the fluid may be induced to flow from the reservoir into the microfluidic device array by the pump. The diagnostic apparatus may further comprise a waste container, that is preferably fluidically coupled to the array outlet channel, for collecting the fluid after flowing through the microfluidic device array. In embodiments, the diagnostic apparatus comprises a heater and/or a cooler, preferably combined with a temperature sensor, for regulating the temperature in the microfluidic device array. In embodiments, the diagnostic apparatus comprises a microcontroller for controlling the various subsystems.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

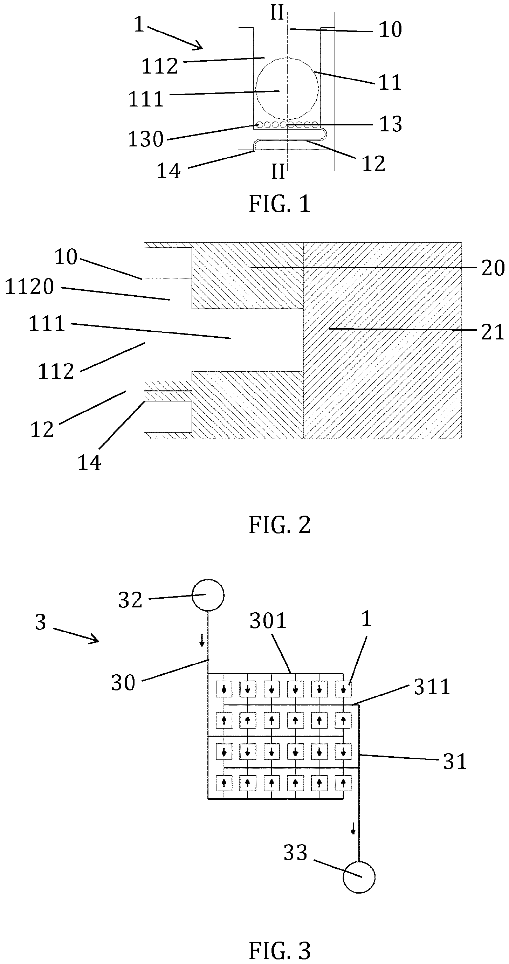

Reference is made to FIG. 1, which is a horizontal cross-section of an example of a microfluidic device unit 1 according to an embodiment of the present invention. In this example, the microfluidic device unit 1 comprises a unit inlet 10 and a unit outlet 14, a cavity 11 comprising a fluidic channel 112, and a well 111 fluidically coupled to the fluidic channel 112. The microfluidic device unit 1 further comprises a filter 13 and a fluidic resistor 12. In this example, the width and the height of the fluidic channel 112 are identical to the width and the height, respectively, of the unit inlet 10, but this is not necessary for the present invention. In this example, the well 111 is cylindrical, with a diameter that is slightly smaller than the width of the fluidic channel 112. Advantageously, because the well 111 is a recess in a wall of the fluidic channel 112, any material comprised in the well 111 is not present in a flow path of fluid flowing between the unit inlet 10 and the unit outlet 14, so that the material in the well 111 may be at least partially prevented from being dragged along by the fluid towards the unit outlet 14. The filter 13 is positioned such that any material in the well 111 dragged along by the fluid is filtered by the filter 13. In this example, the filter 13 comprises a plurality of cylindrical micropillars 130, that is, eight cylindrical micropillars 130, arranged along a straight line. This is however not essential for the invention, and instead, the micropillars 130 may be arranged along a curved line, or arranged along an irregular line. In this example, the distance between neighbouring micropillars 130 of the filter 13 is smaller than the width of the fluidic resistor 12, and is furthermore constant, which is a preferred embodiment of the present invention. The width of the filter 13 is, in this example, identical to the width of the fluidic channel 112, so that fluid may not be able to move towards the fluidic resistor 12 without being filtered by the filter 13. In this example, the fluidic resistor 12 comprises a trench. The fluidic resistor 12 in this example is curved, so that the length of the fluidic resistor 12 may be larger than when the fluidic resistor 12 were straight, which is advantageous as the fluidic resistance of the fluidic resistor 12 is proportional to the length of the fluidic resistor 12. In this example, fluid may be introduced in the unit inlet 10 of the microfluidic device unit 1. The fluid subsequently moves through the fluidic channel 112 of the cavity 11, through the filter 13, through the fluidic resistor 12, and through the unit outlet 14 out of the microfluidic device unit 1. In this example, the unit outlet 14 has the same width as the fluidic resistor 12, but the invention is not limited thereto.

Reference is made to FIG. 2, which is part of a vertical cross-section of an example of a microfluidic device unit 1 according to an embodiment of the present invention, wherein the cross-section is taken along the intermittent line II-II in FIG. 1. The well 111 is a recess in a wall 1120 of the fluidic channel 112, fluidically coupled to the fluidic channel 112. The microfluidic device unit 1 is comprised in a substrate 20, in this example consisting of silicon. The substrate 20 is covered by a cover 21, which in this example consists of glass. Thereby, the cover 21 is an optical window. A wall of the well 111, hence a wall of the cavity, thereby comprises an optical window. The microfluidic device unit 1 is closed on the left by the substrate 20 (not shown) or a sealing layer (not shown).

Reference is made to FIG. 3, which is a diagrammatic illustration of a microfluidic device array 3 according to an embodiment of the present invention, comprising a plurality of microfluidic device units 1 according to an embodiment of the present invention. The microfluidic device array 3 further comprises an array inlet channel 30, comprising, in this example, three array inlet channel branches 301, fluidically coupled to a unit inlet of each of the plurality of microfluidic device units 1. The microfluidic device array 3 further comprises an array outlet channel 31, comprising, in this example, two array outlet channel branches 311, fluidically coupled to a unit outlet of each of the plurality of microfluidic device units 1. Fluid may be introduced in the array inlet channel 30 via the microfluidic device array inlet 32. After flowing through the array inlet channel 30, the microfluidic device units 1, and the array outlet channel 31, the fluid may be removed from the array outlet channel 31 the microfluidic device array outlet 33. Arrows in FIG. 3 indicate the direction of the flow of fluid within the microfluidic device array 3.

It is to be understood that although preferred embodiments, specific constructions and configurations, as well as materials, have been discussed herein for devices according to the present invention, various changes or modifications in form and detail may be made without departing from the scope and spirit of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A microfluidic device unit (1) comprising:
a. a unit inlet (10) and a unit outlet (14),
b. a cavity (11) comprising a fluidic channel (112),
c. a fluidic resistor (12), and
d. a filter (13),
wherein the unit inlet (10), the unit outlet (14), the fluidic channel (112), and the fluidic resistor (12) are fluidically coupled to one another, wherein the cavity (11), the fluidic resistor (12), and the filter (13) are between the unit inlet (10) and the unit outlet (14),
wherein the cavity (11) is upstream of the fluidic resistor (12), and wherein the filter (13) is positioned so as to filter fluid after it enters the fluidic channel (112) and before it enters the fluidic resistor (12).

2. The microfluidic device unit (1) according to claim 1, wherein the filter (13) comprises a plurality of micropillars (130), wherein the distance between neighbouring micropillars (130) is less than or equal to the width of the fluidic resistor (12) and preferably between 50 to 80% of the width of the fluidic resistor (12).

3. The microfluidic device unit (1) according to claim 2, wherein the diameter of the micropillars (130) is from1 to 100 µm, preferably 1 to 20 µm.

4. The microfluidic device unit (1) according to any of the previous claims, wherein the fluidic resistor (12) has a width at least ten times smaller than the width of the fluidic channel (112).

5. The microfluidic device unit (1) according to any of the previous claims, wherein the fluidic resistor (12) has a width of from 1 to 20 µm, preferably from 2 to 10 µm.

6. The microfluidic device unit (1) according to any of the previous claims, wherein the cavity (11) comprises a well (111) fluidically coupled to the fluidic channel (112) and wherein the filter (13) is positioned after the well (111) and before the fluidic resistor (12).

7. The microfluidic device unit (1) according to any of the previous claims, wherein a wall of the cavity (11) comprises an optical window.

8. The microfluidic device unit (1) according to any of the previous claims, wherein the cavity (11) comprises a probe for interacting with an analyte.

9. The microfluidic device unit (1) according to claim 8, wherein the probe is capable of emitting light after interaction with the analyte.

10. A microfluidic device array (3) comprising:
a. an array inlet channel (30) and an array outlet channel (31), and
b. a plurality of microfluidic device units (1) according to any of the previous claims, wherein the unit inlet (10) of each of the microfluidic device units (1) is fluidically coupled to the array inlet channel (30), and wherein the unit outlet (14) of each of the microfluidic device units (1) is fluidically coupled to the array outlet channel (31).

11. A method for sensing an analyte, comprising:
a. obtaining a microfluidic device array (3) according to claim 10,
b. introducing, via the array inlet channel (30) of the microfluidic device array (3), a fluid comprising an analyte, and
c. detecting a response of a probe, comprised in the cavity (11) of a microfluidic device unit (1) comprised in the microfluidic device array (3), to the analyte.

12. The method according to claim 11, wherein detecting a response comprises detecting luminescence emitted by the probe.

13. A process for manufacturing a microfluidic device array (3) according to claim 10, comprising the steps of:
a. providing a substrate (20) comprising the plurality of microfluidic device units (1),
b. providing a cover (21),
c. providing an array inlet channel (30) and an array outlet channel (31) and
d. covering the substrate (20) with the cover (21),
wherein the microfluidic device array (3) is arranged so that the unit inlet (10) of each of the microfluidic device units (1) is fluidically coupled to the array inlet channel (30), and that the unit outlet (14) of each of the microfluidic device units (1) is fluidically coupled to the array outlet channel (31).

14. The process according to claim 13, wherein the cover (21) comprises an optical window.

15. A diagnostic apparatus comprising the microfluidic device array (3) according to claim 10.
